# EUROPÄISCHE PATENTANMELDUNG

(11) **EP 1 127 565 A2**
(43) Veröffentlichungstag der Anmeldung: **29.08.2001**
(21) Anmeldenummer: 01100942.0
(22) Anmeldetag: 17.01.2001
(51) Int. Cl.: A61K 7/06

(54) **Gelförmige Zusammensetzungen und verdickte Haarbehandlungsmittel**

(30) Priorität: 22.02.2000 DE 10007947
(71) Anmelder: Wella Aktiengesellschaft, 64274 Darmstadt (DE)
(72) Erfinder: Lede, Michael, 63225 Langen (DE); Birkel, Susanne, Dr., 95496 Glashütten (DE); Franzke, Michael, Dr., 64380 Rossdorf (DE); Grünkemeier, Miriam, 65343 Eltville (DE); Henze, Hildegard, 64297 Darmstadt (DE)

(57) **Zusammenfassung**

Es werden ein Gel sowie verdickte Haarbehandlungsmittel beschrieben mit einem Gehalt an einer Kombination aus (A) mindestens einem Terpolymeren aus Vinylpyrrolidon, Vinylcaprolactam und einem basischen Acrylamidmonomer und (B) einem Gelbildner ausgewählt aus Gelbildnern, bei denen die Viskosität einer sie enthaltenden Zusammensetzung durch den Zusatz der Komponente (A) erhöht oder maximal um 40% erniedrigt wird. Bevorzugte Gelbildner sind Acrylsäurepolymere, Xanthan Gum oder Hydroxyalkylcellulosen.

## Beschreibung

Gegenstand der Erfindung ist ein Gel mit einem Gehalt an einer Kombination aus einem bestimmten Terpolymeren und ausgewählten Gelbildnern sowie verdickte Haarbehandlungsmittel mit einem Gehalt an der genannten Kombination.

Um dem menschlichen Haar Festigung und Halt zu geben oder um eine erstellte Frisur zu stabilisieren, werden Haarbehandlungsmittel u.a. in Form von verdickten Präparaten wie z.B. verdickten Festigerlotionen, Gelen, Flüssig-Gelen, Sprüh-Gelen etc. eingesetzt. Derartige Produkte enthalten in der Regel eine Kombination aus Gelbildnern und haarfestigenden Polymeren. Die für diese Zwecke üblicherweise verwendeten kosmetischen, haarfestigenden Polymere zeigen in wässrigen, alkoholischen oder wässrig-alkoholischen Medien gute Festigungseigenschaften, die nach der Anwendung mehr oder weniger gut die Haare in Form halten und festigen und die erstellte Frisur stabilisieren. Häufig ist damit aber ein starrer, unnatürlicher Griff des Haares verbunden und die Elastizität der Polymerfilme oder der polymervernetzten Frisur ist ungenügend.

Aus der WO 96/19971 sind Terpolymere aus Vinylpyrrolidon, Vinylcaprolactam und 3-(N-Dimethylaminopropyl)-methacrylamid bekannt sowie deren Verwendung in haarfestigenden Mitteln, insbesondere in Aerosol- und Pumpsprays. Diese Polymere sind besonders für einen Einsatz in wasserhaltigen Sprayrezepturen mit einem reduzierten Gehalt an leicht flüchtigen organischen Bestandteilen (low VOC-Sprays) geeignet. Die Polymere haben gute festigende Eigenschaften, haben aber den Nachteil, dass sie bei einer Verwendung in gelförmigen Zusammensetzungen in Kombination mit einer Reihe von üblicherweise eingesetzten Verdickern die Viskosität der Zusammensetzung deutlich herabsetzen. In diesen Fällen muss daher entweder die Konzentration des verwendeten Verdickers deutlich erhöht werden, was aus Kosten- und anwendungstechnichen Gründen ungünstig ist, oder es muss mit einer weniger geeigneten, d.h. dünnflüssigeren Konsistenz vorlieb genommen werden. Es bestand daher die Aufgabe, die genannten Nachteile zu vermeiden.

Es wurde nun gefunden, daß die Aufgabe gelöst wird durch ein Gel mit einem Gehalt an einer Kombination aus
(A) mindestens einem Terpolymeren aus Vinylpyrrolidon, Vinylcaprolactam und einem basischen Acrylamidmonomer und
(B) einem Gelbildner ausgewählt aus Gelbildnern, bei denen die Viskosität einer sie enthaltenden Zusammensetzung durch den Zusatz der Komponente (A)
   erhöht oder maximal um 40%, vorzusgweise maximal um 30% erniedrigt wird, sowie durch verdickte Haarbehandlungsmittel mit einem Gehalt an dieser Kombination. Das Terpolymer (A) wird vorzugsweise in einer Menge von 0,01 bis 20, besonders bevorzugt von 0,05 bis 10, ganz besonders bevorzugt von 0,1 bis 5 Gew.% und der Gelbildner (B) in einer Menge von 0,01 bis 20, besonders bevorzugt von 0,05 bis 10, ganz besonders bevorzugt von 0,1 bis 5 Gew.% eingesetzt.

Geeignete Terpolymere (A) sind solche, bei denen das Acrylamidmonomer ausgewählt ist aus Dialkylaminoalkylmethacrylamid und Dialkylaminoalkylacrylamid, wobei die Alkylgruppen aus 1 bis 4 Kohlenstoffatomen bestehen. Besonders bevorzugt ist Dimethylaminopropylmethacrylamid. Die Herstellung eines derartigen Polymers wird in der WO 96/19971 beschrieben und ist unter der Bezeichnung Aquaflex® SF 40 (ISP) im Handel erhältlich (INCl-Bezeichnung: PVP/Vinyl Caprolactam/DMAPA Acrylates Copolymer).

Geeignete Gelbildner (B) sind beispielsweise Carbomere (Acrylsäurepolymere), insbesondere solche vom Typ Acrisint® 400, Xanthan Gum oder Hydroxyalkylcellulosen wie z.B. Hydroxyethyl- oder Hydroxypropylcellulose.

In einer bevorzugten Ausführungsform enthält das erfindungsgemäße Haarbehandlungsmittel zusätzlich 0,01 bis 15 Gew.%, vorzugsweise 0,5 bis 10 Gew.% mindestens eines filmbildenden, haarfestigenden oder haarpflegenden Polymers. Dieses Polymer kann nichtionisch, anionisch, kationisch, zwitterionisch oder amphoter sein. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit in Alkohol oder Wasser/Alkohol-Gemischen besitzen, um in dem erfindungsgemäßen Mittel in vollständig gelöster Form vorzuliegen. Unter filmbildenden Polymeren werden erfindungsgemäß solche Polymere verstanden, die bei Anwendung in 0,01 bis 5%iger wässriger, alkoholischer oder wässrig-alkoholischer Lösung in der Lage sind, auf dem Haar einen Polymerfilm abzuscheiden.

Geeignete synthetische, nichtionische filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/Polypropylenglykol Copolymere, die beispielsweise unter den Handelsbezeichnungen Ucon® der Union Carbide vertrieben werden. Besonders bevorzugt sind Polyvinylpyrrolidon und Polyvinylpyrrolidon/Vinylacetat Copolymere.

Geeignete anionische filmbildende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin, Monoethanolamin oder Tetrahydroxypropylethylendiamin und Ammoniak, NaOH und andere. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete Polymere mit Säuregruppen sind insbesondere Copolymere der Acrylsäure oder Methacrylsäure mit Monomeren ausgewählt aus Acrylsäureoder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte Polymere mit Säuregruppen sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere geeignete anionische Polymere sind zum Beispiel Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Geeignete filmbildende amphotere Polymere, sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren bestehend aus Acrylsäure, Methacrylsäure oder deren Ester, wie sie zum Beispiel unter dem Handelsnamen Amphomer® oder Amphomer® LV-71 der Firma NATIONAL STARCH, USA erhältlich sind. Weitere Beispiele für als Komponente (B) geeignete Copolymere mit Säuregruppen sind Copolymere von Acrylsäure, Methylacrylat und Methacrylamidopropyltrimethylammoniumchlorid (INCI-Bezeichnung: Polyquaternium-47), wie sie beispielswiese von der Firma Calgon unter der Handelsbezeichnung Merquat® 2001 vertrieben werden, Copolymere aus Acrylamidopropyltrimethylammoniumchlorid und Acrylaten, wie sie beispielsweise von der Firma Stockhausen unter der Handelsbezeichnung W 37194 erhältlich sind oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43), wie sie beispielsweise von der Firma Societe Francaise Hoechst unter der Handelsbezeichnung Bozequat® 4000 vertrieben werden. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCl-Bezeichnung Methacryloyl Ethyl Betaine/Acrylates Copolymer. Geeignet sind auch die zwitterionischen amphoteren Polymere, die in der JP 10-29919 oder in der JP 10-25344 beschrieben sind.

Geeignete kationische Polymere sind Polymere mit kationischen oder basischen, d.h. kationisierbaren Gruppen. Diese Polymere enthalten stickstoffhaltige Gruppen wie zum Beispiel primäre, sekundäre oder tertiäre Amine. Die basische Gruppe ist entweder in der Polymerkette oder vorzugsweise als Substituent an einem oder mehreren Monomeren enthalten. Das Polymer mit basischen Gruppen kann ein natürliches oder ein synthetisches Homo- oder Copolymer mit aminsubstituierten Monomereinheiten sowie gegebenenfalls mit nicht-basischen Comonomeren sein. Geeignete Polymere mit basischen Gruppen sind zum Beispiel Copolymere von aminsubstituierten Vinylmonomeren und nicht aminsubstituierten Monomeren. Aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise niedere Alkylgruppen wie zum Beispiel C1- bis C7-Alkylgruppen, besonders bevorzugt C1- bis C3-Alkylgruppen sind.

Nicht aminsubstituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Maleinsäureanhydrid, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit kationischen Gruppen enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren Monomeren enthalten sind. Die Ammoniumgruppen enthaltenden Monomere können mit den oben genannten nicht aminsubstituierten Monomeren copolymerisiert sein. Geeignete ammoniumsubstituierte Vinylmonomere sind zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16), quaternisiertes Vinylpyrrolidon/Dimethylaminoethylmethacrylat Copolymer (Polyquaternium-11), Homo- und Copolymere von Dimethyldiallylammoniumchlorid (Polyquaternium-6 und -7), quaternisierte Hydroxyethylcellulose (Polyquaternium-10) oder quaternisierte Guarderivate.

Von den kationischen filmbildenden Polymeren, die in dem erfindungsgemäßen Mittel enthalten sein können, ist zum Beispiel Polyvinylpyrrolidon/Dimethylaminoethylmethacrylat Copolymer geeignet. Weitere kationische Polymere sind beispielsweise das Copolymer aus Polyvinylpyrrolidon und Imidazoliminmethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcaprolactam, quaternierte Ammoniumsalze aus Hydroxyethylcellulose und einem trimethylammonium-substituierten Epoxid und Vinylpyrrolidon/Methacrylamidopropyltrimethylammoniumchlorid Copolymere.

Das erfindungsgemäße Mittel wird bevorzugt in einem wässrigen, einem alkoholischen oder in einem wässrig-alkoholischen Medium mit vorzugsweise mindestens 10 Gew.% Wasser und vorzugsweise maximal 40 Gew.% Ethanol konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Das erfindungsgemäße Mittel kann in einem pH-Bereich von 2,0 bis 9,5 vorliegen. Besonders bevorzugt ist der pH-Bereich zwischen 5 und 8. Als zusätzliche Co-Solventien können organische Lösungsmittel oder ein Gemisch aus Lösungsmitteln mit einem Siedepunkt unter 400°C in einer Menge von 0,1 bis 15 Gew.% bevorzugt von 1 bis 10 Gew.% enthalten sein. Besonders geeignet als zusätzliche Co-Solventien sind Glycerin, Ethylenglykol und Propylenglykol in einer Menge bis 20 Gew.

Das erfindungsgemäße Mittel kann darüber hinaus die für Haarbehandlungsmittel üblichen Zusatzbestandteile enthalten, zum Beispiel Netzmittel oder Emulgatoren aus den Klassen der nichtionischen, anionischen, kationischen oder amphoteren oberflächenaktiven Tenside, wie Fettalkoholsulfate, Alkylbenzolsulfonate, Alkyltrimethylammoniumsalze, Alkylbetaine, in einer Menge von 0,1 bis 15 Gew.%; Feuchthaltemittel; Parfümöle in einer Menge von 0,1 bis 1 Gew.%; Trübungsmittel, wie zum Beispiel Ethylenglykoldistearat, in einer Menge von etwa 0,2 bis 5,0 Gew.%; Perlglanzmittel, wie zum Beispiel ein Gemisch aus Fettsäuremonoalkylolamid und Ethylenglykoldistearat, in einer Menge von etwa 1,0 bis 10 Gew.%; bakterizide und fungizide Wirkstoffe wie zum Beispiel 2,4,4-Trichlor-2-hydroxydiphenylether oder Methylchlorisothiazolion, in einer Menge von 0,01 bis 1,0 Gew.%; Verdickungsmittel, wie beispielsweise Kokosfettsäurediethanolamid, in einer Menge von etwa 0,2 bis 3,0 Gew.%, Puffersubstanzen, wie beispielsweise Natriumcitrat oder Natriumphosphat, in einer Menge von 0,1 bis 1,0 Gew.%; Anfärbestoffe, wie zum Beispiel Fluorescein Natriumsalz, in einer Menge von etwa 0,1 bis 1,0 Gew.%; Pflegestoffe, wie zum Beispiel Pflanzen- und Kräuterextrakte, Protein- und Seidenhydrolysate, Lanolinderivate, in einer Menge von 0,1 bis 5 Gew.%; physiologisch verträgliche Silikonderivate, wie zum Beispiel flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 4 Gew.%; Fettalkohole, Glanzgeber, Vitamine, Weichmacher, Kämmbarkeitsverbesserer, rückfettende Agenzien und Entschäumer.

Das erfindungsgemäße Mittel kann in verschiedenen Applikationsformen Anwendung finden, wie beispielsweise als Gel, Flüssig-Gel, Sprüh-Gel, als verdickte Lotion oder als Gelcreme bzw. als eingetrübtes Gel, wobei eine Anwendung als klares Gel bevorzugt ist. Wenn das erfindungsgemäße Haarbehandlungsmittel in Form eines Haargels vorliegt, so beträgt die Viskosität des Gels vorzugsweise von 500 bis 50.000 cSt, besonders bevorzugt von 1.000 bis 15.000 cSt gemessen als dynamische Viskositätsmessung mit einem HAAKE Rotationsviskosimeter bei einer Temperatur von 25°C, Messkörper Haake SV DIN, Einstellung 8, Geschwindigkeit 5.

Die nachfolgenden Beispiele sollen den Gegenstand der Erfindung näher erläutern. Die in den Beispielen angegebenen Polymergehalte beziehen sich jeweils auf den Feststoffgehalt.

### Beispiele

### Beispiel 1: Vergleichsversuche

Es wurden die Viskositäten gemessen für rein wässrige Zusammensetzungen mit einem Gehalt an verschiedenen Gelbildnern mit und ohne Zusatz von 5 Gew.% Aquaflex ® SF 40 (40%ige Lösung von PVP/Vinyl Caprolactam/DMAPA Acrylates Copolymer in Ethanol). Die Bedingungen für die Viskositätsmessungen waren: Temperatur: 25°C

### Messgerät: HAAKE Rotationsviskosimeter" Messkörper Haake SV DIN, Einstellung 8, Geschwindigkeit 5.

Die Ergebnisse sind in Tabelle 1 zusammengefasst.

**Tabelle 1:**

| Ergebnisse der Viskositätsmessungen | | | |
|---|---|---|---|
| Zusammensetzung | Viskosität ohne Aquaflex® SF40 [Pa s] | Viskosität mit Aquaflex® SF40 [Pa s] | Δ [%] |
| Carbopol® 980, 0,35 Gew.% AMP 0,26 Gew.% | 13,5 | 3,5 | -74 |
| Acrisint® 400 0,6 Gew.% AMP 0,5 Gew.% | 22,5 | 17 | -24 |
| Pemulen® TR1¹⁾ 0,13 Gew% AMP 0,16 Gew.% | 2,9 | 0,744 | -74 |
| Xanthan Gum 2 Gew.% | 1,2 | 2,3 | +92 |
| Karaya Gum 2 Gew.% | 2,08 | 0,028 | -99 |
| Lambda Carrageenan 2 Gew.% | 2,3 | 1,3 | -43 |
| Structure® 3001²⁾ 10 Gew.% | 10,1 | 5,3 | -48 |
| Hydroxyethylcellulose 2 Gew.% | 6,15 | 6,68 | +9 |
| Structure® Plus³⁾ 30 Gew.% Ameisensäure 0,25 Gew.% | > 100 | 13,7 | < -87 |
| Synthalen® W 2000⁴⁾ 6,5 Gew.%; AMP 0,645 Gew.% | 1,8 | 1,1 | -39 |

| | | | |
|---|---|---|---|
| ¹⁾ Acrylates/C10-30 Alkyl Acrylates Crosspolymer | | | |
| ²⁾ Acrylates/Ceteth-20 Itaconate Copolymer | | | |
| ³⁾ Acrylates/Aminoacrylates Copolymer | | | |
| ⁴⁾ Acrylates/C12-14 Pareth-25 Acrylate Copolymer | | | |

In den folgenden Beispielrezepturen bezieht sich die für den Rohstoff Aquaflex ® SF 40 angegebene Menge jeweils auf den Feststoffgehalt des Polymers.

**Beispiel 2:**

| Flüssig-Haargel | |
|---|---|
| 1,0 g | Aquaflex ® SF 40 (PVP/Vinyl Caprolactam/DMAPA Acrylates Copolymer) |
| 1,5 g | Dow Corning Surfactant 193 (Dimethicone Copolyol) |
| 0,3 g | Acrisint® 400 |
| 0,3 g | Aminomethylpropanol 95% |
| 0,15 g | Parfüm |
| 0,2 g | PEG-40 Hydrogenated Castor Oil |
| 0,2 g | PPG-1-PEG-9 Lauryl Glycol Ether |
| 0,4 g | Hydroxyethylcellulose |
| 16,5 g | Ethanol |
| Ad 100 g | Wasser |

**Beispiel 3:**

| Schnell trocknendes Haar-Gel | |
|---|---|
| 2,8 g | Aquaflex ® SF 40 (PVP/Vinyl Caprolactam/DMAPA Acrylates Copolymer) |
| 1,5 g | Dow Corning Surfactant 193 (Dimethicone Copolyol) |
| 0,8 g | Acrisint® 400 |
| 0,62 g | Aminomethylpropanol 95% |
| 0,15 g | Parfüm |
| 0,3 g | Panthenol |
| 34,2 g | Ethanol |
| Ad 100 g | Wasser |

**Beispiel 4:**

| Flüssig-Haargel | |
|---|---|
| 1,0 g | Aquaflex ® SF 40 (PVP/Vinyl Caprolactam/DMAPA Acrylates Copolymer) |
| 1,0 g | Dow Corning Surfactant 193 (Dimethicone Copolyol) |
| 2,5 g | Klucel® HF (Hydroxypropylcellulose) |
| 0,1 g | Zitronensäure |
| 0,15 g | Parfüm |
| 16,5 g | Ethanol |
| Ad 100 g | Wasser |

**Beispiel 5:**

| Haargel | |
|---|---|
| 1,05 g | Aquaflex ® SF 40 (PVP/Vinyl Caprolactam/DMAPA Acrylates Copolymer) |
| 4,2 g | Sorbitol |
| 0,3 g | Acrisint® 400 |
| 1,6 g | Aminomethylpropanol 95% |
| 0,2 g | Parfüm |
| 1,5 g | PEG-25 PABA |
| 0,2 g | Phenoxyethanol |
| 0,05 g | Mica |
| 34,2 g | Ethanol |
| Ad 100 g | Wasser |

**Beispiel 6:**

| Haargel | |
|---|---|
| 3,0 g | Aquaflex ® SF 40 (PVP/Vinyl Caprolactam/DMAPA Acrylates Copolymer) |
| 5,2 g | Glycerin |
| 4,0 g | Propylenglykol |
| 0,35 g | Carbomer |
| 0,3 g | Aminomethylpropanol 95% |
| 0,2 g | Parfüm |
| 1,5 g | Polysorbate-40 |
| 0,2 g | Phenoxyethanol |
| 0,5 g | PEG-25 PABA |
| 4,5 g | Ethanol |
| Ad 100 g | Wasser |

**Beispiel 7:**

| Flüssig-Haargel | |
|---|---|
| 0,5 g | Aquaflex ® SF 40 (PVP/Vinyl Caprolactam/DMAPA Acrylates Copolymer) |
| 7,0 g | Glucose |
| 3,8 g | Propylenglykol |
| 0,3 g | Carbomer |
| 0,3 g | Aminomethylpropanol 95% |
| 0,15 g | Parfüm |
| 0,18 g | PEG-40 Hydrogenated Castor Oil |
| 0,18 g | PPG-1-PEG-9 Lauryl Glycol Ether |
| 0,2 g | Phenoxyethanol |
| 0,5 g | PEG-25 PABA |
| 16,5 g | Ethanol |
| Ad 100 g | Wasser |

## Patentansprüche

1. Gel mit einem Gehalt an einer Kombination aus
(A) mindestens einem Terpolymeren aus Vinylpyrrolidon, Vinylcaprolactam und einem basischen Acrylamidmonomer und
(B) einem Gelbildner ausgewählt aus Gelbildnern, bei denen die Viskosität einer sie enthaltenden Zusammensetzung durch den Zusatz der Komponente (A) erhöht oder maximal um 40% erniedrigt wird.

2. Gel nach Anspruch 1, dadurch gekennzeichnet, daß das basische Acrylamidmonomer ausgewählt ist aus Dialkylaminoalkylmethacrylamid und Dialkylaminoalkylacrylamid, wobei die Alkylgruppen aus 1 bis 4 Kohlenstoffatomen bestehen.

3. Gel nach Anspruch 1 oder 2, dadurch gekennzeichnet, daß der Gelbildner ausgewählt ist aus Gelbildnern, bei denen die Viskosität einer sie enthaltenden Zusammensetzung durch den Zusatz der Komponente (A) erhöht wird.

4. Gel mit einem Gehalt an einer Kombination aus
(A) mindestens einem Terpolymeren aus Vinylpyrrolidon, Vinylcaprolactam und einem basischen Acrylamidmonomer und
(B) einem Gelbildner ausgewählt aus Acrylsäurepolymeren, Xanthan Gum oder Hydroxyalkylcellulosen.

5. Verdicktes Haarbehandlungsmittel mit einem Gehalt an einer Kombination gemäß einem der Ansprüche 1 bis 4.

6. Haarbehandlungsmittel nach Anspruch 5, dadurch gekennzeichnet, daß es zusätzlich mindestens ein weiteres filmbildendes, haarfestigendes oder haarpflegendes Polymer enthält.
